# EUROPEAN PATENT SPECIFICATION

(11) **EP 2 790 759 B1**
(45) Date of publication and mention of the grant of the patent: **19.04.2017**
(21) Application number: 12798752.7
(22) Date of filing: 11.12.2012
(51) Int. Cl.: A61M 15/00

(54) **ASSEMBLY FOR AN INHALATION DEVICE**
ANORDNUNG FÜR EINE INHALATIONSVORRICHTUNG
ENSEMBLE POUR DISPOSITIF D'INHALATION

(30) Priority: 12.12.2011 EP 11193067; 27.08.2012 EP 12181886
(43) Date of publication of application: 22.10.2014
(73) Proprietor: Sanofi SA, 1214 Vernier (CH)
(72) Inventor: MAYER, Stefan, 79098 Freiburg im Breisgau (DE)
(74) Representative: Zwicker, Jörk
(86) International application number: PCT/EP2012/075097
(87) International publication number: WO 2013/087633

(56) References cited:
- EP-A1- 2 201 977
- EP-A1- 2 253 351
- EP-A2- 1 905 472
- DE-A1-102004 041 524

## Description

This disclosure relates to an assembly for an inhalation device and to the use of a sealing member for mechanical cooperation with a metering element of an inhalation device. The sealing member may be part of the previously mentioned assembly.

An inhalation device is described in document WO 2009/065707 A1, for example.

DE 10 2004 041524 A1 discloses an inhaler for powdered medicinal substances comprising a reservoir for the substance and an at least linearly movable dosing chamber for separating a specific amount of the substance from the reservoir. Each of documents EP 1 905 472 A2 and EP 2 201 977 A1 also discloses assembly for an inhalation device, wherein said assembly comprises a reservoir and a dosing chamber.

It is an object of the present disclosure to provide an assembly for an improved inhalation device, e.g. a more efficient inhalation device. Furthermore, it is an object of the present disclosure to provide an improved inhalation device.
This object may, inter alia, be achieved by the subject matter of the independent claims. Advantageous embodiments and refinements are the subject matter of the dependent claims. However, further advantageous concepts may be disclosed herein besides the ones which are claimed

One aspect relates to an assembly for an inhalation device. The inhalation device may be configured for delivering a plurality of doses of a substance to a patient. The assembly may comprise a storage chamber. The storage chamber may be configured to hold a plurality of doses of the substance. The assembly may further comprise a metering element. The metering element may be configured to be moved, preferably rotated and axially moved, with respect to the storage chamber. The metering element may comprise a metering chamber. The metering chamber may be configured for holding a sub-quantity of the substance. The sub-quantity may be retrieved from the storage chamber by means of the metering chamber when the metering element is moved in a dosing direction with respect to the storage chamber. The assembly may further comprise a sealing member. The sealing member may mechanically cooperate with the metering element. The sealing member may be configured for sealing the metering element. The sealing member may comprise an opening. The opening may be a central opening. The opening may receive the metering element. The metering element may be guided through the sealing member, in particular through the opening, when the metering element is moved with respect to the storage chamber. The sealing member may comprise a main part. The sealing member may further comprise a sealing lip. The main part and the sealing lip may be formed unitarily. The sealing lip and the main part may define a recess. The recess may be arranged between the sealing lip and the main part. The sealing lip may be moveable in the inward and in the outward direction with respect to the main part of the sealing member. The sealing lip may be deflectable into the recess.

The recess may enable the sealing lip to be flexible. Due to the flexibility of the sealing lip, the sealing lip may be able to efficiently seal the metering rod and, thus, the storage chamber. In particular, the sealing lip may be configured to prevent moisture from getting into the storage chamber. The sealing lip may be configured to prevent substance from the storage chamber unintentionally leave the storage chamber via the opening in the sealing member in the direction towards a mouthpiece of the inhalation device.

The degree of flexibility of the sealing lip may be determined by the recess, in particular by a depth and/or a width and/or a radius of curvature of the recess. The dimension of the recess and, thus the flexibility of the sealing lip, are expediently chosen such that the sealing lip can sealingly cooperate with different metering elements, in particular metering elements having different outer diameters of different widths which may arise from manufacturing tolerances. The dimension of the recess and, thus the flexibility of the sealing lip, are expediently chosen such that the sealing lip is in sealing contact with the respective metering element at any position of the metering element with respect to the storage chamber when the metering element and the sealing member mechanically cooperate with one another. In this way, an efficient and reliable sealing of the respective metering element is achieved, irrespective of manufacturing tolerances for the metering elements. Hence, provision of reliable inhalation devices is facilitated, even if the metering elements, e.g. metering rods, applied in different devices have varying dimensions.

A further aspect relates to the use of a sealing member. The sealing member may be used for mechanical cooperation with a metering element of an inhalation device. The sealing member may comprise an opening. The opening may be configured for receiving the metering element. The sealing member may further comprise a main part. The sealing member may further comprise a sealing lip. The sealing lip may be moveable in the inward and in the outward direction with respect to the main part of the sealing member. The sealing lip and the main part may define a recess. The recess may be arranged between the sealing lip and the main part. The sealing lip may be deflectable into the recess. The sealing lip may protrude into the opening.

The recess may enable the sealing lip to be flexible in the inward or the outward direction with respect to the main part. The sealing lip may be flexible such that, in the absence of mechanical cooperation with a metering rod, it protrudes into the opening. Accordingly, due to the sealing lip, the opening, in particular a diameter of the opening, may be reduced. When a metering rod is introduced into the opening, the sealing lip may be moved in the outward direction due to mechanical cooperation with the metering rod. The metering rod, in particular the outer dimension of the metering rod may determine how far the sealing lip is moved in the outward direction. The flexibility of the sealing lip may ensure that the sealing lip can efficiently seal metering rods having different outer diameters or widths arising from manufacturing tolerances. The flexibility of the sealing lip may ensure that the sealing lip is in sealing contact with any of these metering elements, irrespective of their outer dimension or width. In this way, an efficient inhalation device is provided.

According to an advantageous embodiment, an assembly for an inhalation device is provided, wherein the assembly comprises
- a storage chamber configured to hold a plurality of doses of a substance,
- a metering element configured to be moved with respect to the storage chamber, the metering element comprising a metering chamber, wherein a sub-quantity of the substance can be retrieved from the storage chamber by means of the metering chamber when the metering element is moved in a dosing direction with respect to the storage chamber, and
- a sealing member mechanically cooperating with the metering element, wherein the sealing member comprises an opening which receives the metering element, and wherein the sealing member comprises a main part and a sealing lip, wherein the sealing lip and the main part define a recess, wherein the recess is arranged between the sealing lip and the main part and, wherein the sealing lip is deflectable into the recess.

According to an advantageous embodiment, the use of a sealing member for mechanical cooperation with a metering element of an inhalation device is described. The sealing member comprises an opening configured for receiving the metering element. The sealing member comprises a main part and a sealing lip, wherein the sealing lip and the main part define a recess, wherein the recess is arranged between the sealing lip and the main part, wherein the sealing lip is deflectable into the recess and, wherein the sealing lip protrudes into the opening.

Of course, features described above in connection with different aspects and embodiments may be combined with each other and with features described below.

Further features and refinements become apparent from the following description of the exemplary embodiments in connection with the accompanying figures.
Figure 1 schematically shows a sectional side view of an inhalation device,
Figure 2 schematically shows a sectional side view of a part of the inhalation device of Figure,
Figure 3 schematically shows a sectional side view of a part of the inhalation device of Figure 1,
Figure 4 schematically shows a sectional view of the sealing member,
Figure 5 schematically shows a sectional view of a metering element,
Figure 6 schematically shows a sectional view of a further embodiment of the sealing member,
Figure 7 schematically shows a perspective view of an exemplary sealing member,
Figure 8 schematically shows a sectional view of the sealing member receiving the metering element,
Figure 9 schematically shows a sectional view of a further embodiment of the sealing member.

Like elements, elements of the same kind and identically acting elements may be provided with the same reference numerals in the figures.

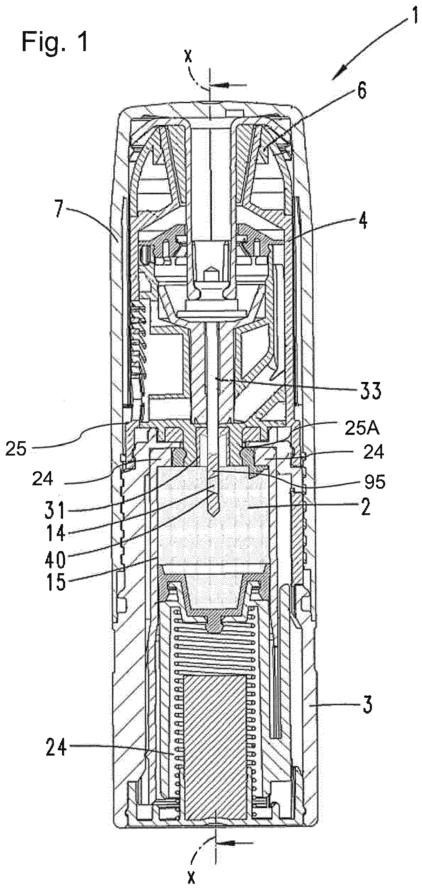

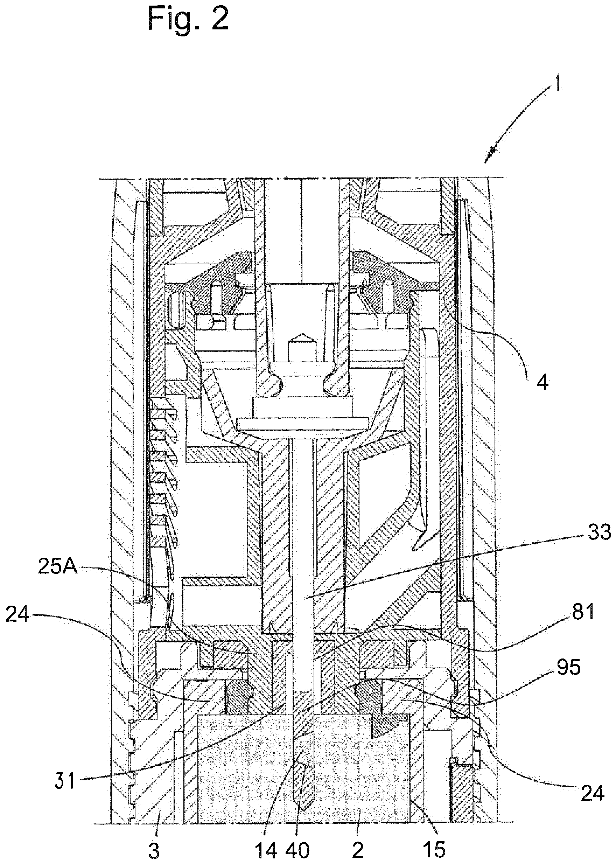

In Figures 1 and 2 an inhalation device 1 or parts of the inhalation device 1 are shown. The inhalation device 1 is configured to be actuated by the suction airflow generated by a user. The inhalation device 1 comprises a housing 3. The device 1 comprises an outer cylinder 4. The outer cylinder 4 is secured against axial movement with respect to the housing 3. The outer cylinder 4 is rotatable with respect to the housing 3.

The inhalation device 1 further comprises a mouthpiece 6. Air is sucked into the device 1 via the mouthpiece 6. The inhalation device 1 comprises a cap 7. The cap 7 is used for covering the mouthpiece 6. The cap 7 may comprise a screw cap. The cap 7 may be rotatable about the main longitudinal axis x of the device 1 in a first direction with respect to the housing 3 for screwing the cap 7 onto the device 1 and in a second direction with respect to the housing 3 for unscrewing the cap 7 from the device 1. The outer cylinder 4 is rotationally connected to the cap 7. In particular, the outer cylinder 4 follows rotation of the cap 7 with respect to the housing 3. For a detailed description of the components of the inhalation device 1 and their mechanical cooperation it is referred to document WO 2009/065707 A1, in particular as far as the operation of the device 1 is concerned.

The device 1 comprises a storage chamber 15. The storage chamber 15 holds one or more doses of a substance 2. The substance may comprise a drug. The substance 2 may comprise a powder.

The storage chamber 15, in particular the section of the storage chamber 15 which is closest to the mouthpiece 6 of the device 1, is terminated by a chamber ceiling 24. The device 1 further comprises a rotary part 25. The rotary part 25 is connected in a rotationally fixed manner to the outer cylinder 4. Accordingly, the rotary part 25 follows rotation of the outer cylinder 4 and, hence, of the cap 7 about the main longitudinal axis x with respect to the storage chamber 15.

The chamber ceiling 24 comprises a central through-opening. A cylindrical portion 25A of the rotary part 25 passes through the central through-opening of the chamber ceiling 24. The lower free end surface of the cylindrical portion 25A is located in the plane of that surface of the chamber ceiling 24 which covers the storage chamber 15. The diameter of the through-opening in the chamber ceiling 24 is larger than the diameter of the cylindrical portion 25A of the rotary part 25.

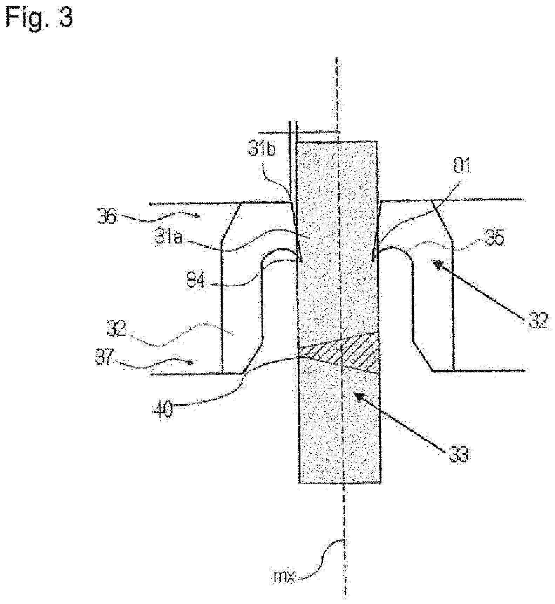

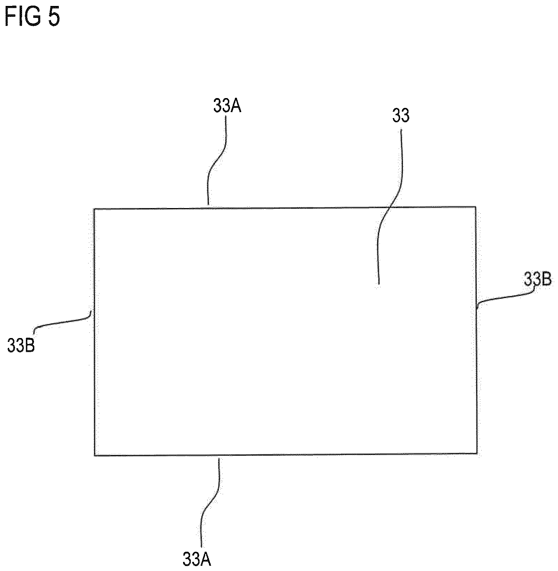

The device 1 further comprises a metering element 33. The metering element 33 may comprise a metering rod. The metering element 33 may have a circular or a non-circular cross-section, such as a rectangular cross-section. As can be gathered from the sectional view of the metering element 33, the metering element 33 may comprise a first or broad side 33A and a second or small side 33B (see Figure 5). The first side 33A may be broader than the second side 33B (see Figure 5). The dimension of the first side 33A may amount to 6 mm, for example. The width or dimension of the first side 33A may be greater than 3 mm and smaller than 10 mm, for example. The dimension of the second side 33B may amount to 1.5 mm, for example. The width or dimension of the second side 33B may be greater than 0.5 mm and smaller than 3 mm, for example. The metering element 33 comprises a main longitudinal axis mx (see Figure 3). The main longitudinal axis mx may coincide with the main longitudinal axis x of the device 1. The metering element 33 is axially and rotationally moveable with respect to the storage chamber 15. In particular, the metering element 33 is moved towards the mouthpiece 6 when the cap 7 is demounted from the device 1, i.e. during an operation of the device 1. This axial direction is in the following referred to as the first axial direction or the dosing direction. The metering element 33 is moved away from the mouthpiece 6 when the cap 7 is re-mounted onto the device 1, i.e. after the operation was completed. This axial direction is in the following referred to as the second axial direction. The metering element 33 is rotationally connected to the rotary part 25 by mechanical cooperation with the rotary part 25. Accordingly, the metering rod 33 follows rotational movement of the cap 7 and, hence, of the rotary part 25 about the main longitudinal axis x when the cap 7 is mounted onto the device 1 or demounted from the device 1.

The metering element 33 comprises a metering chamber 40. The metering chamber 40 is positioned offset from the rotation axis of the metering element 33. The rotation axis is the main longitudinal axis x of the inhalation device 1. The metering element 33 is configured for functioning as a moving metering chamber 40 for accommodating a sub-quantity 14 of the substance 2 which is to be dispensed during the inhalation action performed by a user. The metering chamber 40 is formed in that end section of the metering element 33 which projects into the substance 2. The metering chamber 40 is disposed eccentrically in relation to the side surface of the metering element 33. For detailed description of the operation of the metering element 33, it is referred to document WO 2009/065707 A1.

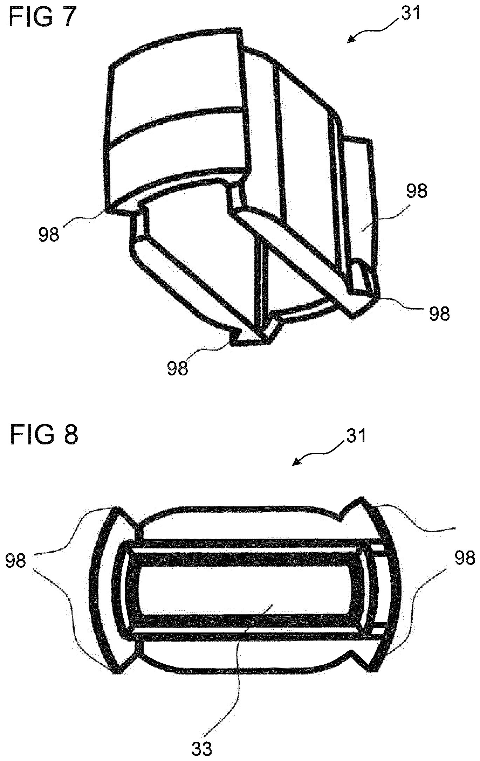

The device 1 further comprises a sealing member 31. The cylindrical portion 25A accommodates the sealing member 31 in its center. In particular, the sealing member 31 is arranged subsequently to the cylindrical portion 25A in the radial inward direction with respect to the housing 3. The sealing member 31 is arranged between the cylindrical portion 25A and the metering element 33. The sealing member 31 is arranged circumferentially around the metering element 33, as can be seen in Figure 8, for example. The sealing member 31 is arranged at that end of the storage chamber 15 which is closest to the mouthpiece 6. The sealing member 31 is arranged outside of the storage chamber 15.

The sealing member 31 comprises or consists of a rubber material or a similar elastic material. The sealing member 31 comprises a thermoplastic elastomer, for example. The sealing member 31 may be produced by a two-component injection molding process together with the rotary part 25. Alternatively, the sealing member 31 may be produced in a separate manufacturing step. In this embodiment, the sealing member 31 and the rotary part 25 are connected to one another such that the sealing member 31 is secured against axial and rotational movement with respect to the rotary part 25. The sealing member 31 may, for example, be clipped into the rotary part 25.

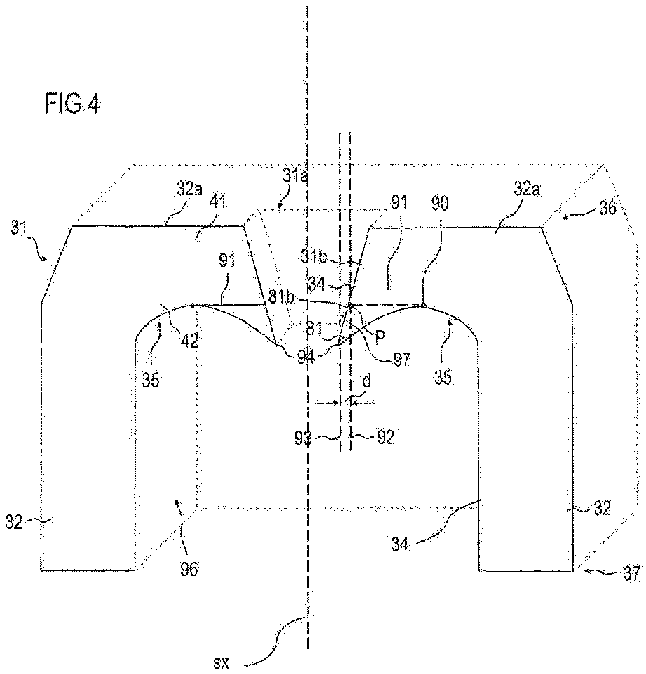

The sealing member 31 may be snap-fitted to the cylindrical portion 25A of the rotary part 25, for example. For this purpose, the sealing member 31 may comprise connection elements 98 (see Figures 7 and 8). The connection elements 98 may be arranged at an outer surface of the sealing member 31. The connection elements 98 may be integrally formed with a main part 32 (see, for example, Figure 4) of the sealing member 31. The connection elements 98 may comprise an indentation and/or a protrusion. The connection elements 98 may be arranged on the outer surface of the sealing member 31 for connecting the sealing member 31 to the cylindrical portion 25A.

The sealing member 31 is configured for sealing that end of the storage chamber 15 which is closest to the mouthpiece 6. The sealing member 31 is configured for sealing the metering element 33. In particular, the sealing member 31 is configured to prevent moisture from getting into the storage chamber 15 via an opening via which the metering element 33 passes through the sealing member 31 into the storage chamber 14. The sealing member 31 is further configured to prevent substance 2 from the storage chamber 15 to get unintentionally across the sealing member 31 via the opening via which the metering element 33 passes through the sealing member 31 into the storage chamber 15. The sealing member 31 is described in detail in connection with Figures 3 and 4.

Figure 3 schematically shows a sectional side view of a part of the inhalation device of Figure 1. Figure 4 schematically shows a sectional view of a part of the sealing member.

In plan view, the sealing member 31 may be cylindrically or rectangularly shaped, which can be seen in the Figures 7 and 8, for example. The sealing member 31 comprises an opening 31 a (see, in particular, Figure 4). The opening 31 a is elongated, preferably rectangular, in plan view onto the opening 31 a. The opening 31 a comprises a dimension which is great enough such that the metering element 33 can be moved through the sealing member 31, in particular through the opening 31 a. In particular, the opening 31 a receives the metering element 33. The opening 31 a may be arranged in the center of the sealing member 31. Accordingly, the opening 31 a may be a central opening.

The sealing member 31 comprises a main part 32. The main part 32 extends in the axial direction from a first end 36 of the sealing member 31 to a second end 37 of the sealing member 31. The first end 36 is that end which is closest to the mouthpiece 6. The second end 37 is the end which is furthest away from the mouthpiece 6.

In the section of the first end 36, the sealing member 31, in particular the main part 32, comprises a protrusion 32a (see, in particular, Figure 4). The protrusion 32a may be as rigid as the main part 32. The protrusion 32a is directed in the inward direction towards the metering element 33. The protrusion 32a is delimited in the inward direction by a side face 31 b. The side face 31 b faces towards the metering element 33. The side face 31 b is oblique with respect to the main longitudinal axis x. The side face 31 b delimits the opening 31 a of the sealing member 31.

In the direction towards the storage chamber 15, in particular in the direction towards the second end 37, the opening 31 a widens up into a cavity 96 of the sealing member 31. The cavity 96 is delimited by an inner surface 34 of the sealing member 31, in particular of the main part 32.

The sealing member 31 comprises a sealing lip 81. The sealing lip 81 is less rigid than the protrusion 32a. The protrusion 32a protrudes from the main part 32 in the inward direction and transitions in the axial direction into the sealing lip 81. The sealing lip 81 and the main part 32 of the sealing member 31 are formed unitarily. The sealing lip 81 is oriented in an axial direction opposite to the first or dosing direction in which the metering element 33 is moved with respect to the storage chamber 15 during operation of the device 1. In other words, the sealing lip 81 is oriented in the second direction with respect to the storage chamber 15. The sealing lip 81 is arranged in the section of the first end 36 of the sealing member 31. The sealing lip 81 is circumferentially disposed along opening 31 a of the sealing member 31. The contour of the sealing lip 81 is adapted to the cross-section of the metering element 33.

The sealing lip 81 and the main part 32 define a recess 35. In particular, the recess 35 is arranged between the sealing lip 81 and the main part 32. The recess 35 is arranged in the section of the first end 36 of the sealing member 31. The recess 35 is defined by the protrusion 32a. The protrusion 32a has a first end section 41 and a second end section 42. The first end section 41 is the section which is closest to the mouthpiece 6 and the second end section 42 is the section which is furthest away from the mouthpiece 6. The recess 35 is arranged in the second end section 42 of the protrusion 32a. The recess 35 faces towards the storage chamber 15. The recess 90 comprises a lowest point 90. The lowest point 90 is that point of the recess 35 which is furthest away from the storage chamber 15. The sealing lip 81 defines a side wall of the recess 35. In particular the sealing lip 81 defines that side wall of the recess 35 which is arranged most inwardly with respect to the housing 3. The contour of the recess 35 is adapted to the cross-section of the metering element 33.

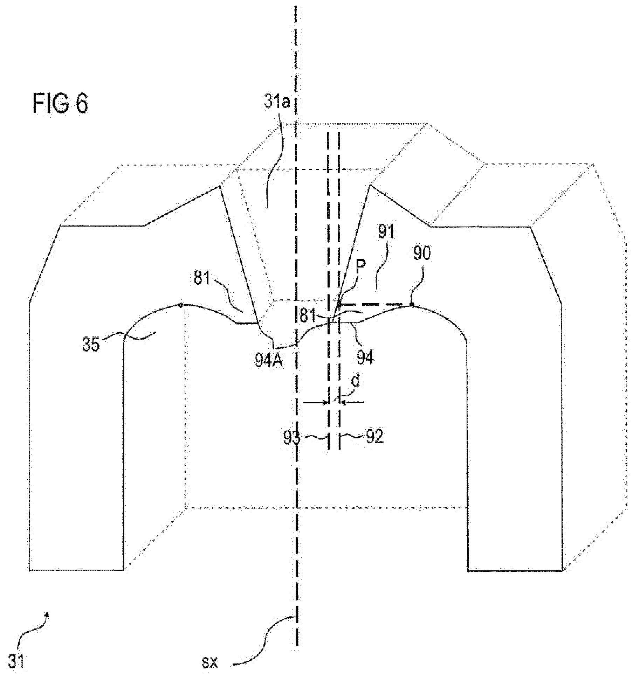

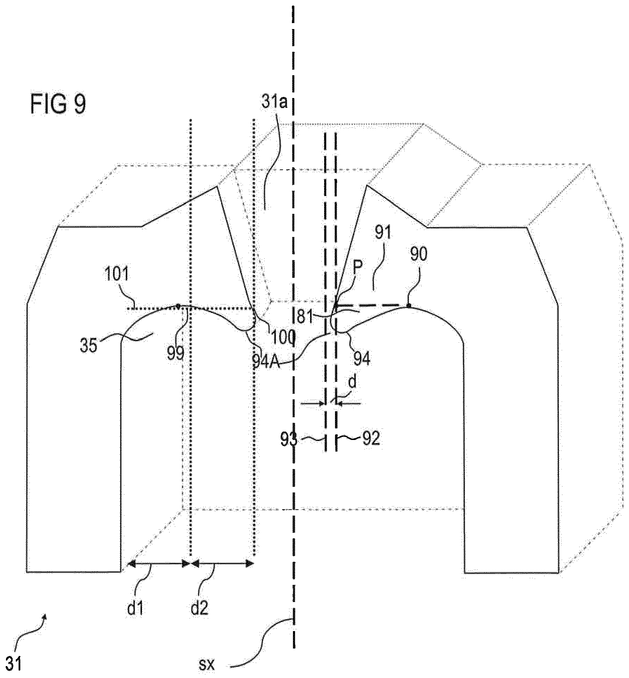

The sealing lip 81 comprises a tip or end 94. The tip 94 is arranged at that end of the sealing lip 81 which is furthest away from the mouthpiece 6. In one embodiment (see Figure 4), the tip or end 94 is sharp-ended. Accordingly, the sealing lip 81 may taper off in the direction of the storage chamber 15, i.e. its thickness reduced. The tip or end 94 may taper-off in a single point. In an alternative embodiment (see Figure 6), the tip or end 94 is flat. In particular, the tip or end 94 does not taper-off in the direction of the storage chamber 15 into a single point as it is the case for the sharp-ended tip 94. Rather, in this embodiment, the tip or end 94 comprises an end-face which may extend perpendicularly to the longitudinal axis sx of the sealing lip 81. The dimension of this end-face may be less than 3 mm and larger than 1 mm, for example. The dimension of the end-face may amount to 2 mm, for example. As compared to the embodiment where the tip or end 94 is sharp-ended, in this embodiment the tip or end 94 may be cut-off. In an alternative embodiment (see Figure 9), the tip or end 94 is rounded. Accordingly, the tip or end 94 does not taper-off in the direction of the storage chamber 15 to an extend as it is the case for the sharp-ended tip 94. Rather, the tip or end 92 may comprise the segment of a circle, for example. In particular, the tip or end 92 may be curved. The tip or end 92 may be significantly more curved than the sharp-ended tip or end 92. The radius of curvature of the tip or end 92 may be between 0.1 mm and 0.2 mm, for example. The radius of curvature of the tip or end 92 may amount to 0.15 mm, for example. By means of a rounded tip or end 92, sharp edges of the sealing lip 81 can be avoided. The sealing lip 81 can thus more easily be slid along the outer surface of the metering element 33 when the metering element 33 is moved with respect to the sealing member 31. The rounded tip or end 92 may comprise a greater material thickness in the radial direction as compared to the sharp-ended tip or end 92.

Accordingly, as compared to the sealing lip 81 comprising the sharp-ended tip or end 92, the sealing lip 81 comprising the rounded tip or end 92 may more easily be prevented from protruding into the metering chamber 40 when the metering element 33 is moved with respect to the sealing lip 81, in particular, when the metering chamber 40 passes the sealing lip 81, which is described later on in more detail. In this way, unintentional removal of substance 2 from the metering chamber 40 may be prevented more effectively as it is the case for the sealing lip 81 with the sharp-ended tip or end 92.
A distance d1 (see Figure 9) between the inner surface 34 of the cavity 96 and a point 99 is between 0.2 mm and 0.3 mm, preferably 0.25 mm. The point 99 marks the onset of the curvature of the rounded tip or end 92 begins. The distance d1 may be such that excess powdery substance 95 wiped off the metering element 33 can be effectively guided back into the storage chamber 15. A distance d2 between the point 99 and a point 100 is between 0.2 mm and 0.3 mm, preferably 0.25 mm. The point 100 is that point where a line 101 which is perpendicular to axis sx and which crosses point 99 crosses the side face 31 b. The distance d2 may indicate the width or radial extension of the rounded tip or end 92. In other words, the width of the rounded tip or end 92 may amount to 0.25 mm, for example.

The sealing lip 81 comprises an interaction face 81 b. The interaction face 81 b points into the inward direction and towards the metering element 33. The sealing lip 81, in particular the interaction face 81 b of the sealing lip 81 mechanically cooperates with the metering element 33. In particular, the metering element 33 slides along the sealing lip 81 when the metering element 33 is moved in the first or in the second direction with respect to the storage chamber 15. The side face 31 b merges into the interaction face 81 b. The interaction face 81 b of the sealing lip 81 is an extension of the side face 31 b. In the absence of mechanical cooperation with the metering element 33 (see Figure 4), the interaction face 81 b is oblique with respect to the main longitudinal axis x. Upon mechanical cooperation with the metering element 33, the interaction face 81 b is less oblique with respect to the main longitudinal axis x as compared to in the absence of mechanical cooperation.

The sealing lip 81 is configured to protrude into the opening 31 a of the sealing member 31. In particular, the sealing lip 81 protrudes as far into the opening 31 a as it is necessary for the sealing lip 81 to get into sealing contact with the metering element 33. This is explained in the following in detail:

The sealing lip 81 is flexible. In particular, the sealing lip 81 is moveable in the inward or the outward direction with respect to the main part 32. Thereby, the sealing lip 81 may be deflected into the recess 35. In other words, the recess 35 enables the sealing lip 81 to be moved in the inward and/or outward direction. The dimensioning of the recess 35, in particular the depth and/or the radial extension of the recess 35, may determine the degree of flexibility of the sealing lip 81. The deeper and wider the recess 35, the more flexible is the sealing lip 81, for example.

The sealing lip 81 must be flexible such that, in a pre-assembly state of the device 1, i.e. a state where the components of the device 1 are not yet assembled, in particular when the metering element 33 is not yet guided through the opening 31 a of the sealing member 31 (see Figure 4), the sealing lip 81 protrudes into the opening 31 a for less than or equal to 0.5 mm. Accordingly, the dimension of the opening 31 a, in particular a diameter of the opening 31 a is reduced due to the sealing lip 81 protruding into the opening 31 a. Preferably, the sealing lip 81 protrudes into the opening 31 a for 0.5 mm. The value of the protrusion of the sealing lip 81 may be defined as follows (see Figure 4):
The sealing member 31 comprises a first longitudinal axis 92. The longitudinal axis 92 is parallel to the main longitudinal axis mx of the metering element 33. The longitudinal axis 92 is parallel to a main longitudinal axis sx of the sealing member 31. The sealing member 31 comprises a second longitudinal axis 93. The second longitudinal axis 93 is parallel to the main longitudinal axis mx of the metering element 33. The second longitudinal axis 93 is parallel to the main longitudinal axis sx of the sealing member 31. The second longitudinal axis 93 crosses the end or tip 94 of the sealing lip 81. In the embodiment where the end of tip 94 is flat (see Figure 6), the axis 93 crosses the most inward directed point 94A of the end or tip 94 of the sealing lip 81. The first longitudinal axis 92 and the second longitudinal axis 93 are arranged at a distance d from one another.

The device 1 comprises a line 91 which extends perpendicularly to the first and second longitudinal axis 92, 93. In particular, the line 91 is perpendicular to the main longitudinal axes sx and mx. The perpendicular line 91 runs through the lowest point 90 of the recess 35. The perpendicular line 91 crosses or runs through the surface of the sealing member 31 within the opening 31 a, in particular it crosses or runs through the side face 31 b, at a certain point P. The first longitudinal axis 92 crosses the perpendicular line 91 at that point P. The distance d between the first longitudinal axis 92 and the second longitudinal axis 93 is that distance for which the sealing lip 81 protrudes into the opening 31 a. Accordingly, the distance d between the first longitudinal axis 92 and the second longitudinal axis 93 is smaller than or equal to 0.5 mm.

In an assembled state of the inhalation device 1 (see Figures 1 to 3), i.e. the state where the components of the device 1 are assembled, in particular where the metering element 33 is guided through the opening 31 a, the sealing lip 81 is then biased in the outward direction with respect to the metering element 33 by mechanical cooperation with the metering element 33. In other words, the metering element 33 forces the sealing lip 81 outwardly and at least partly out of the opening 31 a when the metering rod 33 is introduced into the opening 31 a, the sealing lip 81 thereby deflecting into the recess 35. The sealing lip 81 is, however, not compressed due to mechanical cooperation with the metering element 33, i.e. the volume is not reduced. The distance by which the sealing lip 81 is forced out of the opening 31 a depends on the outer dimension of the respective metering element 33. The sealing lip 81 exerts an inwardly directed force onto the sides 33A, 33B, i.e. onto the first side 33A and onto the second side 33B, of the metering element 33. The force which the sealing lip 81 exerts onto the first side 33A is greater than the force which the sealing lip 81 exerts onto the second side 33B of the metering element 33. The force which the sealing lip 81 exerts onto the first side 33A is constant for any position of the metering element 33 with respect to the sealing lip 81. The force which the sealing lip 81 exerts onto the second side 33B is constant for any position of the metering element 33 with respect to the sealing lip 81. The force which the sealing lip 81 exerts onto the metering element 33 depends on the profile of the sealing lip 81. The profile of the sealing lip 81 is, for example, defined by the dimension of the recess 35, e.g. the depth of the recess 35 and/or the radius of curvature of the recess 35, which is described later on in detail. Additionally or alternatively, the profile may be defined by the dimension of the tip 94 of the sealing lip 81, e.g. by a sharp-ended (see Figure 3) or by a flat tip 94 (see Figure 6). The profile can be adjusted during manufacture of the sealing lip 81, which is described later on in detail.

Due to manufacturing tolerances, different metering elements 33 may comprise different outer dimensions, i.e. different side faces 33A, 33B. In particular, the outer dimensions of different metering elements 33 may differ from one another by up to 1.0 mm, for example. For example, one metering element 33 may comprise a first side 33A which is equal to 6 mm and second side which is equal to 1 mm. A further metering element 33 may comprise a first side 33A which is equal to 5 mm and a second side which is equal to 1.5 mm, for example. The sealing member 31, in particular the sealing lip 81, is configured to compensate said manufacturing tolerances. In particular, the sealing member 31 is adapted to receive and to seal metering elements 33 with any outer dimension or with any width which may arise from the manufacturing process. Thereby, for a metering rod 33 which comprises a larger first side 33A, e.g. having a width of 7.0 mm, as compared to an average first side of about 6 mm, for example, the sealing lip 81 is forced farther in the outward direction with respect to the metering element 33 by mechanical cooperation with the metering element 33. At most, the sealing lip 81 may be forced in the outward direction such that the interaction face 81 b of the sealing lip 81 runs parallel to the first longitudinal axis 92 (not explicitly shown in the Figures). On the other hand, for a metering rod 33 which comprises a smaller first side 33A, e.g. 5 mm, as compared to the average first side 33A of 6 mm, the sealing lip 81 may be deflected only marginally in the outward direction with respect to the metering element 33 by mechanical cooperation with the metering element 33. All in all, due to the flexibility of the sealing lip 81, variations in the outer dimension of different metering elements 33, which variations arise from manufacturing tolerances, can be compensated and the respective metering element 33 can be efficiently sealed by means of the sealing member 31.

For different metering elements 33, in particular metering elements 33 with different outer dimensions, the sealing lip 81 provides a constant force onto the metering element 33, in particular onto the sides 33A, 33B of the metering element 33, as described above. In other words, the sealing lip 81 provides the same force for different outer dimensions of different metering elements 33. As described above, said force onto the sides 33A, 33B of the metering element 33 is constant at any position of the respective metering element 33 with respect to the storage chamber 15 where the sealing member 31, in particular the sealing lip 81, and the metering element 33 cooperate with one another. In this way, powdery substance 2, in particular the sub-quantity 14 of the powdery substance 2, retained in the metering chamber 40 is prevented from dropping out of the metering chamber 40 when the sealing lip 81 mechanically cooperates with the metering chamber 40 when the metering element 33 is moved in the first direction. In other words, when the metering element 33 is moved in the first direction, the metering chamber 40 slides along the sealing lip 81, the sealing lip 81 thereby providing a constant force onto the sides 33A, 33B of the metering element 33 and, thus, onto the metering chamber 40 of the metering element 33. In this way, the sealing lip 81 prevents dropping out of substance 2 from the metering chamber 40 during movement of the metering chamber 40 out of the storage chamber 15.

For being able to compensate the variations described above, in a first step, a plurality of metering elements 33 are manufactured. In a next step, the variation for the distribution of the outer dimension, in particular of the widths of the sides 33A, 33B, with respect to an average value must be determined for each metering element 33 of the plurality of manufactured metering elements 33. In a next step, the sealing lip 81 must be adjusted such that, upon mechanical cooperation with the respective metering element 33, it exerts a constant force onto the sides 33A, 33B of each of the different metering elements 33. For this purpose, the dimension of the recess 35 may be adjusted, in particular the depth and/or the width and/or the radius of curvature of the recess 35 can be adjusted, such that the sealing lip 81 exerts a constant force onto the sides 33A, 33B, of each of the metering elements 33. The depth of the recess 35 may be equal to 0.1 mm, for example. The depth of the recess 35 may be greater than 0.05 mm, for example. The depth of the recess 35 may be less than 0.25 mm, for example. The radius of curvature may amount to 0.2 mm, for example. The radius of curvature may be less than 0.5 mm, preferably less than 0.3 mm. The radius of curvature may be greater than 0.05 mm, preferably greater than 0.1 mm. In a next step, the device 1 is assembled, in particular the metering element 33 is guided through the opening 31 a of the sealing member 31. In particular, for each metering element 33 the sealing member 31 and, in particular, the sealing lip 81 can be adapted and arranged such that the sealing lip 81 is forced in the outward direction when it mechanically cooperates with the metering rod 33. In this way, an efficient and reliable sealing is provided by way of a single sealing member 31 which is adapted to be used with metering elements 33 having different dimensions due to manufacturing tolerances.

It may, furthermore, occur that a fraction of the substance 2, i.e. excess substance 95 (see Figures 1 and 2) sticks to the metering element 33 when it is moved out of the storage chamber 15 into the first direction. This excess substance 95 should not be delivered to the user during an inhalation operation as it exceeds the amount which is intended for a dose, which is confined within the metering chamber 40. Even more, this excess substance 95 could get completely lost for further dose setting and dose delivery operations when the excess substance 95 is moved along with the metering element 33 across the sealing member 31 out of the storage chamber 15. The sealing member 31 helps to avoid that the excess substance 95 gets lost for being delivered to the user. In other words, the sealing member 31 prevents substance 2 from the storage chamber 15 to get unintentionally across the sealing member 31 via the opening 31 a. For this purpose, the recess 35 must be configured such that the sealing member 31 and, in particular the sealing lip 81, is sufficiently deflectable in the inward and outward direction with respect to the metering element 33 for wiping the excess substance 95 off the metering element 33. In particular, the depth and/or the radius of curvature of the recess 35 must be configured such that the excess-substance 95 can be wiped off the metering element 33 when the metering element 33 is moved with respect to the sealing member 31. The dimension of the recess 35 may be adjusted to the degree of flexibility of the sealing lip 81 necessary for wiping off the excess substance 95 and, thus, to the amount of excess powdery substance 95 to be wiped off the metering element 33.

When the metering element 33 is moved in the first direction, it slides along the sealing lip 81. When the metering element 33 and, in particular the sides 33A, 33B, slide along the sealing lip 81, the sealing lip 81 scrapes or wipes the excess substance 95 off the outer surface, in particular off the sides 33A, 33B, of the metering element 33. The excess substance 95 is moved in the outward direction with respect to the sealing lip 81 and into the recess 35. From the recess 35, the excess powdery substance 95 may be guided along the inner surface 34 through the cavity 96 of the sealing member 31 in the second direction with respect to the storage chamber 15 and back into the storage chamber 15. Alternatively, from the recess 35, the excess-substance 95 may fall back onto the surface formed by the substance 2 within the storage chamber 15. Accordingly, the excess powdery substance 95 is moved back into the storage chamber 15 for being available for a subsequent dose setting and dose delivery operation.

Furthermore, when the metering element 33 is moved with the respect to the storage chamber 15, the sealing lip 81 expediently does not protrude into the metering chamber 40. If the sealing lip 81 protruded into the metering chamber 40 the sub-quantity 14 could be unintentionally removed from the metering chamber 40 upon mechanical cooperation with the sealing lip 81.The sealing lip 81 and the recess 35 are configured such that the sealing lip 81 is prevented from protruding into the metering chamber 40 when the metering element 33 is moved with respect to the sealing lip 81, in particular when the metering chamber 40 passes the sealing lip 81. For this purpose, the distance by which the sealing lip 81 protrudes into the opening 31 a in a pre-assembled state as described above should not be greater than 0.5 mm. In addition, the direction of orientation of the sealing lip 81 must be chosen such that the sealing lip 81 does not protrude into the metering chamber 40. In particular, an angle 97 between the interaction face 81 b of the sealing lip 81 and the second longitudinal axis 93 should be less than 5 degrees and greater than 0.5 degree, for example. The angle 92 should amount to 4 degrees, for example. In addition to that, the flexibility of the sealing lip 81 should be chosen so as to prevent the sealing lip 81 from protruding into the metering chamber 40. In particular, the width and/or the depth and/or the radius of curvature of the recess 35 should be chosen such that the sealing lip 81 is efficiently deflected into the recess 35 and does not protrude in the metering chamber 40 when the metering element 30 is moved with respect to the storage chamber 15, in particular when the metering chamber 40 mechanically cooperates with the sealing lip 81. The recess 35 should have a maximum depth, i.e. the depth at the deepest point 90, which is less than 0.25 mm, for example. Preferably, the maximum depth amounts to 0.1 mm. In this way, it is prevented that the sealing lip 81 protrudes into the metering chamber 40. In addition to that, the shape of the tip or end 94 of the sealing lip 81 can be chosen such that the sealing lip 81 is prevented from protruding into the metering chamber 40 when the metering element 33 is moved with respect to the sealing lip 81. The tip or end 94 may be rounded or curved, for example.

The term drug as used herein may mean a pharmaceutical formulation containing at least one pharmaceutically active compound, for example for the treatment of obstructive airway or lung diseases such as asthma or chronic obstructive pulmonary disease (COPD), local respiratory tract oedema, inflammation, viral, bacterial, mycotic or other infection, allergies, diabetes mellitus.

The active pharmaceutical compound is preferably selected from the group consisting of active pharmaceutical compounds suitable for inhalation, preferably antiallergenic, antihistamine, anti-inflammatory, antitussive agents, bronchodilators, anticholinergic drugs, and combinations thereof.

The active pharmaceutical compound may for example be chosen from:
an insulin such as human insulin, e.g. a recombinant human insulin, or a human insulin analogue or derivative, a glucagon-like peptide (GLP-1) or an analogue or derivative thereof, or exendin-3 or exendin-4 or an analogue or derivative of exendin-3 or exendin4;
an adrenergic agent such as a short acting β2-agonists (e.g. Salbutamol, Albuterol, Levosalbutamol, Fenoterol, Terbutaline, Pirbuterol, Procaterol, Bitolterol, Rimiterol, Carbuterol, Tulobuterol, Reproterol), a long acting β2-agonist (LABA, e.g. Arformoterol, Bambuterol, Clenbuterol, Formoterol, Salmeterol), an ultra LABA (e.g. Indacaterol) or another adrenergic agent (e.g. Epinephrine, Hexoprenaline, Isoprenaline (Isoproterenol), Orciprenaline (Metaproterenol));
a glucocorticoid (e.g. Beclometasone, Budesonide, Ciclesonide, Fluticasone, Mometasone, Flunisolide, Betamethasone, Triamcinolone);
an anticholinergic agent or muscarinic antagonist (e.g. Ipratropium bromide, Oxitropium bromide, Tiotropium bromide);
a mast cell stabilizer (e.g. Cromoglicate, Nedocromil);
a xanthine derivative (e.g. Doxofylline, Enprofylline, Theobromine, Theophylline, Aminophylline, Choline theophyllinate);
an eicosanoid inhibitor, such as a leukotriene antagonist (e.g. Montelukast, Pranlukast, Zafirlukast), a lipoxygenase inhibitor (e.g. Zileuton) or a thromboxane receptor antagonist (e.g. Ramatroban, Seratrodast);
a phosphodiesterase type-4 inhibitor (e.g. Roflumilast);
an antihistamine (e.g. Loratadine, Desloratadine, Cetirizen, Levocetirizine, Fexofenadine);
an allergen immunotherapy (e.g. Omalizumab);
a mucolytic (e.g. Carbocisteine, Erdosteine, Mecysteine);
an antibiotic or antimycotic;
or a combination of any two, three or more of the above-mentioned compound classes or compounds (e.g. Budesonide/Formoterol, Fluticasone/Salmeterol, Ipratropium bromide/Salbutamol, Mometasone/Formoterol);
or a pharmaceutically acceptable salt or solvate or esters of any of the above named compounds.

Pharmaceutically acceptable salts are for example acid addition salts and basic salts. Acid addition salts are e.g. a chloride, bromide, iodide, nitrate, carbonate, sulfate, methylsulfate, phosphate, acetate, benzoate, benzenesulfonate, fumarate, malonate, tartrate, succinate, citrate, lactate, gluconate, glutamate, edetate, mesylate, pamoate, pantothenate or a hydroxy-naphthoate salt. Basic salts are for example salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology. Pharmaceutically acceptable ester may for example be acetates, propionates, phosphates, succinates or etabonates.

Pharmaceutically acceptable solvates are for example hydrates.

Other implementations are within the scope of the following claims. Elements of different implementations may be combined to form implementations not specifically described herein.

### Reference numerals

- 1: Inhalation device
- 2: Substance
- 3: Housing
- 4: Outer cylinder
- 6: Mouthpiece
- 7: Cap
- 14: Sub-quantity
- 15: Storage chamber
- 24: Chamber ceiling
- 25: Rotary part
- 25: Cylindrical portion
- 31: Sealing member
- 31a: Opening
- 31b: Side face
- 32: Main part
- 32: Protrusion
- 33: Metering element
- 33A: First side
- 33B: Second side
- 34: Inner surface
- 35: Recess
- 36: First end
- 37: Second end
- 40: Metering chamber
- 41: First end section
- 42: Second end section
- x: Device axis
- 81: Sealing lip
- 84: Proximal end-face
- 81b: Side face
- d: Distance
- 90: Lowest point
- 91: Straight line
- 92: Perpendicular line
- 93: Perpendicular line
- 94: Tip
- 94A: Point
- 95: Excess substance
- 96: Cavity
- 97: Angle
- 98: Connection element
- P: Point
- mx: Longitudinal axis of metering element
- sx: Longitudinal axis of sealing member
- d1: Distance
- d2: Distance
- 99: Point
- 100: Point
- 101: Line

## Claims

1. An assembly for an inhalation device (1), wherein the assembly comprises:
- a storage chamber (15) configured to hold a plurality of doses of a substance (2),
- a metering element (33) configured to be moved with respect to the storage chamber (15), the metering element (33) comprising a metering chamber (40), wherein a sub-quantity (14) of the substance (2) can be retrieved from the storage chamber (15) by means of the metering chamber (40), when the metering element (33) is moved in a dosing direction with respect to the storage chamber (15), and
- a sealing member (31) mechanically cooperating with the metering element (33), wherein the sealing member (31) comprises an opening (31 a) which receives the metering element (33), and wherein the sealing member (31) comprises a main part (32) and a sealing lip (81),
**characterized by**
the sealing lip (81) and the main part (32) defining a recess (35), wherein the recess (35) is arranged between the sealing lip (81) and the main part (32), and wherein the sealing lip (81) is deflectable into the recess (35).

2. The assembly according to claim 1,
wherein the sealing lip (81) is oriented in an axial direction with respect to the storage chamber (15), and wherein the axial direction is opposite to the dosing direction in which the metering element (33) is moved with respect to the storage chamber (15) during a dosing operation for retrieving substance (2) from the storage chamber (15).

3. The assembly according to claim 1 or claim 2,
wherein the sealing lip (81) is configured to protrude into the opening (31 a) of the sealing member (31).

4. The assembly according to claim 3,
wherein the recess (35) faces the storage chamber (15), and wherein the metering element (33) has a longitudinal axis (mx), and wherein the sealing lip (81) is configured to protrude inwardly into the opening (31a) such that a distance (d) between
A) a first longitudinal axis (92) of the assembly which is parallel to the longitudinal axis (mx) of the metering element (33) and which crosses a line (91) perpendicular to the longitudinal axis (mx) of the metering element (33) extending from a lowest point (90) of the recess (35) in the inward direction to a side face (31b) of the sealing member (31) at that point (P) where the perpendicular line (91) meets the side face (31 b) and
B) a second longitudinal axis (93) of the assembly which is parallel to the longitudinal axis (mx) of the metering element (33) and runs through a tip (94) of the sealing lip (81) is smaller than or equal to 0.5 mm.

5. The assembly according to any of the previous claims,
wherein the opening (31 a) is elongated, preferably rectangular, in plan view onto the opening (31 a), and wherein the sealing lip (81) is circumferentially disposed along the opening (31 a).

6. The assembly according to any of the previous claims,
wherein the sealing lip (81) is adapted and arranged for removing excess powdery substance (95) from the metering element (33) when the metering element (33) is moved into the dosing direction with respect to the storage chamber (15) and wherein the recess (35) is configured to receive the excess substance (95).

7. The assembly according to any of the previous claims,
wherein the sealing lip (81) is adapted and arranged for providing a constant force onto the metering element (33) at any position of the metering element (33) with respect to the storage chamber (15) when the sealing member (31) and the metering element (33) mechanically cooperate with one another.

8. The assembly according to any of the previous claims,
wherein the sealing lip (81) is adapted and arranged such that it is prevented from being compressed when the sealing lip (81) mechanically cooperates with the metering element (33).

9. The assembly according to any of claims 4 to 8, wherein the side face (31 b) of the sealing member (31) faces in the inward direction towards the metering element (33), and wherein the sealing lip (81) comprises an interaction face (81 b) facing into the inward direction towards the metering element (33), wherein the interaction face (81b) is adapted and arranged to mechanically cooperate with the metering element (33), wherein the side face (31 b) transitions into the interaction face (81 b) and, wherein the side face (31b) is oblique with respect to the longitudinal axis (mx) of the metering element (33).

10. The assembly according to any of the previous claims,
wherein the sealing lip (81) and the recess (35) are configured such that the sealing lip (81) is prevented from protruding into the metering chamber (40) when the metering chamber (40) passes the sealing lip (81) such that removal of the sub-quantity (14) from the metering chamber (40) upon mechanical cooperation with the sealing lip (81) is prevented.

11. The assembly according to any of the previous claims,
wherein the main part (32) and the sealing lip (81) are formed unitarily.

## Patentansprüche

1. Anordnung für eine Inhalationsvorrichtung (1), wobei die Anordnung Folgendes umfasst:
- eine Vorratskammer (15), die zum Haften einer Vielzahl von Dosen einer Substanz (2) konfiguriert ist,
- ein Dosierelement (33), das dazu konfiguriert ist, bezüglich der Vorratskammer (15) bewegt zu werden, wobei das Dosierelement (33) eine Dosierkammer (40) umfasst, wobei eine Teilmenge (14) der Substanz (2) mittels der Dosierkammer (40) aus der Vorratskammer (15) entnommen werden kann, wenn das Dosierelement (33) in einer Dosierrichtung bezüglich der Vorratskammer (15) bewegt wird, und
- ein Dichtglied (31), das mit dem Dosierelement (33) mechanisch zusammenwirkt, wobei das Dichtglied (31) eine Öffnung (31a) umfasst, die das Dosierelement (33) aufnimmt, und wobei das Dichtglied (31) einen Hauptteil (32) und eine Dichtlippe (81) umfasst,
**dadurch gekennzeichnet, dass**
die Dichtlippe (81) und der Hauptteil (32) eine Aussparung (35) definieren, wobei die Aussparung (35) zwischen der Dichtlippe (81) und dem Hauptteil (32) angeordnet ist und wobei die Dichtlippe (81) in die Aussparung (35) ablenkbar ist.

2. Anordnung nach Anspruch 1,
wobei die Dichtlippe (81) in einer axialen Richtung bezüglich der Vorratskammer (15) ausgerichtet ist und wobei die axiale Richtung der Dosierrichtung entgegengesetzt ist, in die das Dosierelement (33) bezüglich der Vorratskammer (15) zur Entnahme von Substanz (2) aus der Vorratskammer (15) während eines Dosiervorgangs bewegt wird.

3. Anordnung nach Anspruch 1 oder Anspruch 2,
wobei die Dichtlippe (81) dazu konfiguriert ist, in die Öffnung (31 a) des Dichtglieds (31) zu ragen.

4. Anordnung nach Anspruch 3,
wobei die Aussparung (35) der Vorratskammer (15) zugewandt ist und wobei das Dosierelement (33) eine Längsachse (mx) hat und wobei die Dichtlippe (81) dazu konfiguriert ist, nach innen in die Öffnung (31a) zu ragen, so dass ein Abstand (d) zwischen
A) einer ersten Längsachse (92) der Anordnung, die parallel zu der Längsachse (mx) des Dosierelements (33) verläuft und eine Linie (91) kreuzt, die senkrecht zu der Längsachse (mx) des Dosierelements (33) verläuft und sich von einem untersten Punkt (90) der Aussparung (35) in der Einwärtsrichtung zu einer Seitenfläche (31b) des Dichtglieds (31) an dem Punkt (P) erstreckt, an dem die senkrechte Linie (91) auf die Seitenfläche (31 b) trifft, und
B) einer zweiten Längsachse (93) der Anordnung, die parallel zu der Längsachse (mx) des Dosierelements (33) und durch eine Spitze (94) der Dichtlippe (81) verläuft,
kleiner als oder gleich 0,5 mm ist.

5. Anordnung nach einem der vorhergehenden Ansprüche, wobei die Öffnung (31a) in Draufsicht auf die Öffnung (31a) länglich und vorzugsweise rechteckig ist und wobei die Dichtlippe (81) umfangsmäßig entlang der Öffnung (31 a) angeordnet ist.

6. Anordnung nach einem der vorhergehenden Ansprüche, wobei die Dichtlippe (81) dazu geeignet und angeordnet ist, dass überschüssige pulverförmige Substanz (95) aus dem Dosierelement (33) entfernt wird, wenn das Dosierelement (33) in der Dosierrichtung bezüglich der Vorratskammer (15) bewegt wird, und wobei die Aussparung (35) zur Aufnahme der überschüssigen Substanz (95) konfiguriert ist.

7. Anordnung nach einem der vorhergehenden Ansprüche, wobei die Dichtlippe (81) dazu geeignet und angeordnet ist, dass sie das Dosierelement (33) in einer beliebigen Position des Dosierelements (33) bezüglich der Vorratskammer (15) mit einer konstanten Kraft beaufschlagt, wenn das Dichtglied (31) und das Dosierelement (33) mechanisch miteinander zusammenwirken.

8. Anordnung nach einem der vorhergehenden Ansprüche, wobei die Dichtlippe (81) dazu geeignet und angeordnet ist, dass verhindert wird, dass sie komprimiert wird, wenn die Dichtlippe (81) mechanisch mit dem Dosierelement (33) zusammenwirkt.

9. Anordnung nach einem der Ansprüche 4 bis 8, wobei die Seitenfläche (31 b) des Dichtglieds (31) in der Einwärtsrichtung zum Dosierelement (33) hin weist und wobei die Dichtlippe (81) eine Zusammenwirkungsfläche (81b) umfasst, die in der Einwärtsrichtung zum Dosierelement (33) hin weist, wobei die Zusammenwirkungsfläche (81b) dazu geeignet und angeordnet ist, mechanisch mit dem Dosierelement (33) zusammenzuwirken, wobei die Seitenfläche (31 b) in die Zusammenwirkungsfläche (81 b) übergeht und wobei die Seitenfläche (31 b) bezüglich der Längsachse (mx) des Dosierelements (33) schräg ist.

10. Anordnung nach einem der vorhergehenden Ansprüche,
wobei die Dichtlippe (81) und die Aussparung (35) so konfiguriert sind, dass die Dichtlippe (81) daran gehindert wird, in die Dosierkammer (40) zu ragen, wenn die Dosierkammer (40) an der Dichtlippe (81) vorbeigeht, so dass ein Entfernen der Teilmenge (14) aus der Dosierkammer (40) beim mechanischen Zusammenwirken mit der Dichtlippe (81) verhindert wird.

11. Anordnung nach einem der vorhergehenden Ansprüche,
wobei der Hauptteil (32) und die Dichtlippe (81) einheitlich ausgebildet sind.

## Revendications

1. Ensemble pour un dispositif d'inhalation (1), l'ensemble comprenant :
- une chambre de stockage (15) configurée pour renfermer une pluralité de doses d'une substance (2),
- un élément de mesure (33) configuré pour être déplacé par rapport à la chambre de stockage (15), l'élément de mesure (33) comprenant une chambre de mesure (40), une sous-quantité (14) de la substance (2) pouvant être extraite de la chambre de stockage (15) par le biais de la chambre de mesure (40), lorsque l'élément de mesure (33) est déplacé dans une direction de dosage par rapport à la chambre de stockage (15), et
- un organe d'étanchéité (31) coopérant de manière mécanique avec l'élément de mesure (33), l'organe d'étanchéité (31) comprenant une ouverture (31a) qui reçoit l'élément de mesure (33), et l'organe d'étanchéité (31) comprenant une partie principale (32) et une lèvre d'étanchéité (81),
**caractérisé en ce que**
la lèvre d'étanchéité (81) et la partie principale (32) définissent un creux (35), le creux (35) étant disposé entre la lèvre d'étanchéité (81) et la partie principale (32), et la lèvre d'étanchéité (81) pouvant être courbée dans le creux (35).

2. Ensemble selon la revendication 1,
dans lequel la lèvre d'étanchéité (81) est orientée dans une direction axiale par rapport à la chambre de stockage (15), et dans lequel la direction axiale est opposée à la direction de dosage dans laquelle l'élément de mesure (33) est déplacé par rapport à la chambre de stockage (15) lors d'une opération de dosage visant à extraire de la substance (2) de la chambre de stockage (15).

3. Ensemble selon la revendication 1 ou la revendication 2,
dans lequel la lèvre d'étanchéité (81) est configurée pour faire saillie dans l'ouverture (31a) de l'organe d'étanchéité (31).

4. Ensemble selon la revendication 3,
dans lequel le creux (35) fait face à la chambre de stockage (15), et dans lequel l'élément de mesure (33) présente un axe longitudinal (mx), et dans lequel la lèvre d'étanchéité (81) est configurée pour faire saillie vers l'intérieur dans l'ouverture (31a) de telle sorte qu'une distance (d) entre
A) un premier axe longitudinal (92) de l'ensemble qui est parallèle à l'axe longitudinal (mx) de l'élément de mesure (33) et qui croise une ligne (91) perpendiculaire à l'axe longitudinal (mx) de l'élément de mesure (33), s'étendant d'un point le plus bas (90) du creux (35) dans la direction allant vers l'intérieur jusqu'à une face latérale (31b) de l'organe d'étanchéité (31), au niveau du point (P) où la ligne perpendiculaire (91) rencontre la face latérale (31 b) et
B) un second axe longitudinal (93) de l'ensemble qui est parallèle à l'axe longitudinal (mx) de l'élément de mesure (33) et s'étend à travers une pointe (94) de la lèvre d'étanchéité (81)
soit inférieure ou égale à 0,5 mm.

5. Ensemble selon l'une quelconque des revendications précédentes, dans lequel l'ouverture (31 a) est allongée, de préférence rectangulaire, en vue en plan sur l'ouverture (31a), et dans lequel la lèvre d'étanchéité (81) est disposée de manière circonférentielle le long de l'ouverture (31a).

6. Ensemble selon l'une quelconque des revendications précédentes, dans lequel la lèvre d'étanchéité (81) est conçue et disposée pour éliminer un excès de substance pulvérulente (95) de l'élément de mesure (33) lorsque l'élément de mesure (33) est déplacé dans la direction de dosage par rapport à la chambre de stockage (15) et dans lequel le creux (35) est configuré pour recevoir l'excès de substance (95).

7. Ensemble selon l'une quelconque des revendications précédentes, dans lequel la lèvre d'étanchéité (81) est conçue et disposée pour appliquer une force constante sur l'élément de mesure (33) dans n'importe quelle position de l'élément de mesure (33) par rapport à la chambre de stockage (15) lorsque l'organe d'étanchéité (31) et l'élément de mesure (33) coopèrent l'un avec l'autre de manière mécanique.

8. Ensemble selon l'une quelconque des revendications précédentes, dans lequel la lèvre d'étanchéité (81) est conçue et disposée de telle sorte qu'elle ne puisse pas être comprimée lorsque la lèvre d'étanchéité (81) coopère de manière mécanique avec l'élément de mesure (33).

9. Ensemble selon l'une quelconque des revendications 4 à 8,
dans lequel la face latérale (31b) de l'organe d'étanchéité (31) est orientée dans la direction allant vers l'intérieur en direction de l'élément de mesure (33), et dans lequel la lèvre d'étanchéité (81) comprend une face d'interaction (81 b) orientée dans la direction allant vers l'intérieur en direction de l'élément de mesure (33), dans lequel la face d'interaction (81b) est conçue et disposée pour coopérer de manière mécanique avec l'élément de mesure (33), dans lequel la face latérale (31b) devient la face d'interaction (81b) et dans lequel la face latérale (31b) est oblique par rapport à l'axe longitudinal (mx) de l'élément de mesure (33).

10. Ensemble selon l'une quelconque des revendications précédentes, dans lequel la lèvre d'étanchéité (81) et le creux (35) sont configurés de telle sorte que la lèvre d'étanchéité (81) ne puisse faire saillie dans la chambre de mesure (40) lorsque la chambre de mesure (40) passe la lèvre d'étanchéité (81) de telle sorte que le retrait de la sous-quantité (14) depuis la chambre de mesure (40) lors d'une coopération mécanique avec la lèvre d'étanchéité (81) ne soit pas possible.

11. Ensemble selon l'une quelconque des revendications précédentes, dans lequel la partie principale (32) et la lèvre d'étanchéité (81) sont réalisées d'un seul tenant.
